# EUROPEAN PATENT SPECIFICATION

(11) **EP 3 901 134 B1**
(45) Date of publication and mention of the grant of the patent: **03.08.2022**
(21) Application number: 20210296.8
(22) Date of filing: 27.11.2020
(51) Int. Cl.: C07C 303/02, C07C 303/22, C07C 303/32, C07C 303/44, C07C 309/12

(54) **METHOD AND SYSTEM FOR PREPARING HIGH-PURITY TAURINE AND SALT**
VERFAHREN UND SYSTEM FÜR DIE HERSTELLUNG VON HOCHREINEM TAURIN UND SALZ
PROCÉDÉ ET SYSTÈME DE PRÉPARATION DE TAURINE ET DE SEL DE HAUTE PURETÉ

(30) Priority: 22.04.2020 CN 202010320728
(43) Date of publication of application: 27.10.2021
(73) Proprietor: Qiangjiang Yongan Pharmaceutical Co., Ltd., Qianjiang, Hubei 433100 (CN)
(72) Inventor: Chen, Yong, Qianjiang, Hubei, 433100 (CN); Fang, Xiquan, Qianjiang, Hubei, 433100 (CN); Li, Shaobo, Qianjiang, Hubei, 433100 (CN); Liu, Feng, Qianjiang, Hubei, 433100 (CN)
(74) Representative: Gulde & Partner

(56) References cited:
- CN-A- 110 483 342

## Description

### Field

The present disclosure relates to a production method in a process for chemically synthesizing taurine, in particular to a method for producing high-purity taurine and salt by an ethylene oxide method, and a production system thereof.

### Background

Taurine has a chemical name of 2-aminoethanesulfonic acid, and is sulfur-containing free amino acid which is the richest in content in body cells. A chemical synthesis process route of the taurine mainly includes an ethylene oxide method and an ethanolamine method, wherein preparation of the ethylene oxide method includes three steps of:
(1) by using ethylene oxide as a starting material, performing an addition reaction on the ethylene oxide and sodium bisulfate to obtain sodium hydroxyethyl sulfonate;
   wherein the main reaction is that:

   *CH*₂*CH*₂*O* + *NaHSO*₃ → *HOCH₂CH*₂*SO*₃*Na*
(2) carrying out ammonolysis on sodium hydroxyethyl sulfonate to obtain sodium taurate; and

   *HOCH*₂*CH*₂*SO*₃*Na* + *NH₃ → H*₂*NCH*₂*CH*₂*SO*₃*Na* + *H*₂*O*
(3) adding sulfuric acid for acidification to obtain the taurine and the salt.

2*H*₂*NCH*₂*CH*₂*SO*₃*Na* + *H*₂*SO*₄ → 2*H*₂*NCH*₂*CH*₂*SO*₃*H* + *Na*₂*SO*₄

The Chinese Patents CN101508657, CN10158658, CN10158659 and CN101486669 and patent application CN 110 483 342 A describe a method of obtaining taurine and sodium sulfate by neutralizing sodium taurate with sulfuric acid. After cooling, by filtering a crystal suspension, a taurine crude product can be very easily obtained. However, the waste mother solution still contains the taurine, ammonium sulfate and other organic impurities. Those patents also provide feasible methods for further separating these components from the waste solution so as to implement economy of production and reduce discharge of wastes. However, all materials are mixed in the same mother solution, and thus, a high difficulty in separation between the taurine, sulfate and the like is caused, the process design is complex, energy is simultaneously extracted, cost of labor and the like is very high, and meanwhile, product purities of the taurine and the sulfate are relatively low.

The US Patents US9428450B2 and US9428451B2 and the European Patent EP3133060B1 introduce a method for circularly producing taurine, and describe a principle and method of separating the taurine from sodium sulfate in detail. Solubility of the sodium sulfate is the maximum at a temperature of 33 DEG C. At a temperature between 33 DEG C and 100 DEG C, the solubility of the sodium sulfate is gradually and slightly reduced, but within a range of 0 DEG C to 33 DEG C, the solubility of the sodium sulfate is sharply reduced. At a temperature of 40 DEG C or above, the sodium sulfate is separated out in a form of crystals without crystal water; and at a temperature of 30 DEG C or below, the sodium sulfate is separated out in a form of mirabilite (i.e., sodium sulfate decahydrate). Each time when the taurine is extracted, the temperature is controlled within a range of about 33 DEG C, the taurine is extracted, and meanwhile, sulfate is prevented from being crystallized; then the mother solution obtained after extraction is evaporated and crystallized; and the sodium sulfate is separated at a temperature of 70 DEG C to 95 DEG C. The method mainly utilizes different solubility characteristics of the sulfate and the taurine at different temperatures to implement separation between the taurine and the sodium sulfate; the operation process recorded in the method is very complex; several materials are in the same mother solution system, so purities of the products obtained by separation are necessarily relatively low.

CN110683970A introduces a method for removing a sodium sulfate solid impurity from taurine. The method mainly is that the taurine is extracted from sodium sulfate by adopting a novel deep-eutectic solvent preparation method, a deep-eutectic solvent is formed by utilizing monoethanolamine and the taurine, the sodium sulfate impurity is removed by filtering, and separation is carried out by utilizing a series of means of organic solvent reextraction and the like so as to obtain a pure taurine elementary substance. Such method introduces many types of organic solvents, and is also relatively cumbersome to operate; an intermediate control point needs to be very accurate, and the industrialization difficulty is very high.

CN109020839A introduces a recycling process for preparing taurine by carrying out ammonolysis on sodium hydroxyethyl sulfonate, the content of a taurine crude product is 90%, and sodium sulfate is transformed into a water glass product with low requirements for impurities, and an effect of solving a problem of processing the sodium sulfate is achieved. The process method has the main problem that the process method cannot solve the purity problem of the taurine crude product and the sodium sulfate and is just a subsequent processing process for low-content low-purity products.

From the above, the existing process for preparing the taurine by a sulfuric acid neutralization method still has many defects in the aspect of separation and purification of the taurine and the sulfate, which are mainly shown in a complex separation process, low purities of separated products, a low extraction rate and high extraction cost. Therefore, it is urgent to develop a process capable of preparing high-purity taurine and sulfate or other salts for solving the problems above.

### Summary

In order to overcome the defects in the prior art, the present disclosure aims to provide a method and system for preparing high-purity taurine and salt, which are simple in process and low in production cost.

In order to fulfill the aim of the present disclosure, the technical solution provided by the present disclosure is as follows: a method for preparing high-purity taurine and salt includes the following steps of:
according to the method for preparing the high-purity taurine and salt, reacting ethylene oxide with bisulfite to generate isethionate, carrying out an ammonolysis reaction on the isethionate and ammonia as well as a metal salt, carrying out evaporation to obtain a taurine salt concentrated solution, subjecting the concentrated solution to ion exchange in an ion exchange system to obtain an adsorption solution with a main ingredient of taurine, separately collecting the adsorption solution, and extracting the taurine from the adsorption solution; and eluting adsorbed metal cations by an acid, separately collecting the eluate, and extracting the salt from the eluate or directly using the eluate as a salt solution product.

Wherein, the bisulfite is sodium bisulfite, ammonium bisulfite, potassium bisulfite or lithium bisulfate, and preferably the sodium bisulfite and the ammonium bisulfate. The metal salt is any one or a mixture of any two or more of sodium hydroxide, potassium hydroxide, lithium hydroxide, sodium carbonate, potassium carbonate, lithium carbonate, sodium sulfate, potassium sulfate and lithium sulfate, and preferably is the sodium hydroxide or the sodium sulfate. The acid is sulfuric acid, hydrochloric acid, phosphoric acid, water-soluble carboxylic acid or sulfonic acid, and preferably is the sulfuric acid and the hydrochloric acid.

In the ion exchange system, an adsorption unit and an elution unit of the ion exchange system are respectively cleaned with purified water, and correspondingly obtained cleaning water is respectively collected.

In an adsorbing state, when pH of a solution at an outlet of the adsorption unit is 4 to 10, the adsorption solution is collected; and in an eluting state, when pH of a solution at an outlet of the elution unit is 2 to 8, the eluate is collected.

Metal cations which are at least equivalent in amount to residual isethionate anions are allowed to enter the adsorption solution.

Preferably, in terms of the taurine, the concentration of the concentrated solution entering the ion exchange system is 10% to 35%.

Preferably, the concentration of the acid added into the ion exchange system is 5% to 35%.

Preferably, an adsorbent of the ion exchange system is ion exchange resin; and the ion exchange resin is resin with a function that the cations can be adsorbed.

Preferably, the taurine extraction process of the adsorption solution includes the steps of: evaporation concentration, cooling crystallization and solid-liquid separation, cooling crystallization and solid-liquid separation are carried out at 5 DEG C to 30 DEG C, and a taurine crude product obtained by separation has a content of over 95% and a purity of 98.5% or above.

Preferably, the salt is extracted from the eluate in an evaporation crystallization mode, wherein the evaporation crystallization is carried out at 60 DEG C to 125 DEG C, and a salt crude product obtained by separation has a content of over 97% and a purity of 98.5% or above.

On the basis of the preparation method, the present disclosure further provides a system for preparing high-purity taurine and salt, which is used for a process for producing the taurine by an ethylene oxide method and includes an addition reaction device, an ammonolysis reaction device, an evaporation device and a taurine salt concentrated solution collection device. The ammonolysis reaction device is provided with a metal salt inlet, the taurine salt concentrated solution collection device is connected with an ion exchange system for ion exchange, the ion exchange system is provided with an acid inlet, an adsorption solution outlet and an eluate outlet, the adsorption solution outlet is connected with a taurine extraction device, and the eluate outlet is connected with a salt extraction device; and the ion exchange system is provided with a purified water inlet, an adsorption unit cleaning water outlet and an elution unit cleaning water outlet.

The cleaning water can be efficiently recycled, and preferably, the adsorption unit cleaning water outlet is connected with the taurine salt concentrated solution collection device, and the elution unit cleaning water outlet is connected with the salt extraction device.

From the above, the present disclosure relates to the method for producing the high-purity taurine and salt. Materials of two target products are efficiently and simply separated in an ion exchange mode after the ammonolysis reaction, strict separation of material systems is implemented, and moreover, extraction and purification are separately carried out, so that two target products of the taurine and the salt have very good crystal forms of crystallization, a final extraction rate is greatly promoted, and the purities of the obtained taurine and salt are both very high, wherein the taurine crude product can reach the content of 95% or above and the purity of over 98.5%, the salt crude product can reach the content of 97% or above and can reach the purity of 98.5% or above, and after recrystallization, the content of both the products is 99.5% or above.

In a conventional taurine production process, by taking a byproduct sulfate as an example, the taurine and the sulfate exist in the same mother solution system, separation has to be carried out by utilizing the solubility difference between the taurine and the sulfate, i.e., when solubility of sodium sulfate is the maximum, firstly, the taurine is extracted, and then a solution containing a great amount of taurine and sulfate after extraction is subjected to concentration separation; generally, firstly, the sulfate is separated out at a high temperature, then cooling is carried out to a temperature of about 33 DEG C to extract the taurine, and extraction is repeatedly carried out for many times; and the process is very cumbersome to operate, and an extraction rate and product quality of the taurine are also seriously restricted. Obviously, all the materials exist in the same system and the materials are high in viscosity, resulting a poor crystal form of crystallization of the sulfate, and thus, the sodium sulfate obtained by separation is relatively low in purity; meanwhile, it is inevitable that the taurine crude product contains a great amount of sulfate, and meanwhile, it is also inevitable that the sulfate contains the taurine, resulting in a difficulty of separating the taurine from the mother solution, and thus, the extracted taurine is relatively low in content and the yield is reduced.

Compared to the conventional production process, the preparation method disclosed by the present disclosure is equivalent to extracting the taurine from the high-purity taurine mother solution system and extracting the salt from the high-purity salt mother solution system, and is completely not related to interference brought to product separation when two target products exist in the same maternal system, so that the processes for extracting two target products are simplified, the production cost is greatly reduced, and further, the automatic, intelligent and continuous design is also easier to implement in the whole product production process.

### Brief Description of the Drawings

In order to clearly illustrate the technical solution of the embodiments of the disclosure, the drawings of the embodiments will be briefly described one by one in the following; and it is obvious that the described drawings are only related to some embodiments of the disclosure, and a person of ordinary skill in the art also can obtain other drawings, without any inventive work, according to the drawings.
Fig. 1 is a flow chart of a process for preparing high-purity taurine and salt products in an application example of the present disclosure;
Fig. 2 is a flow chart of a process for recycling cleaning water of an ion exchange system in an application example of the present disclosure;
Fig. 3 is a flow chart of a process for extracting a taurine product from an adsorption solution in an application example of the present disclosure;
Fig. 4 is a flow chart of a process for extracting a sulfate product froman eluatein an application example of the present disclosure;
Fig. 5 is a schematic diagram of a production system for preparing high-purity taurine and salt products in an application example of the present disclosure; and
Fig. 6 is a structural schematic diagram of single resin column in an application example of the present disclosure.

### Detailed Description of the Embodiments

The specific contents of the present disclosure will be further described in detail below in combination with the accompanying drawings and specific embodiments,

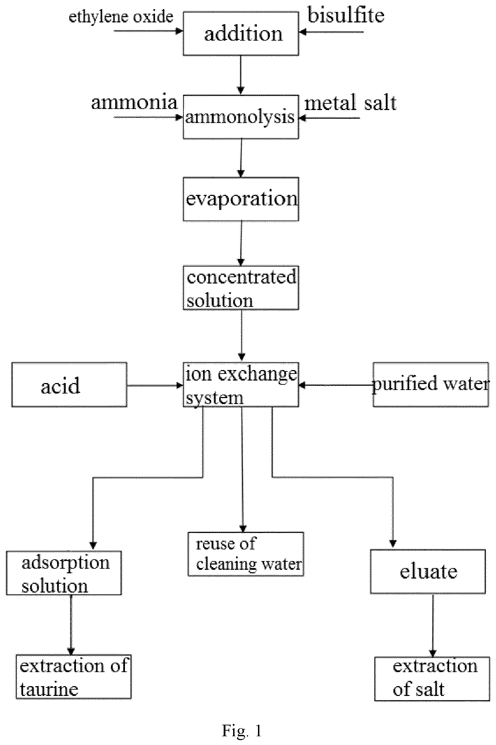

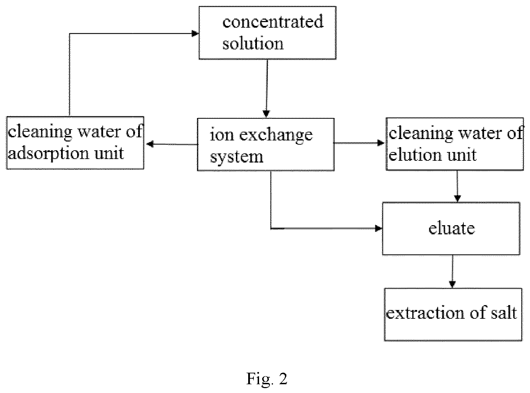

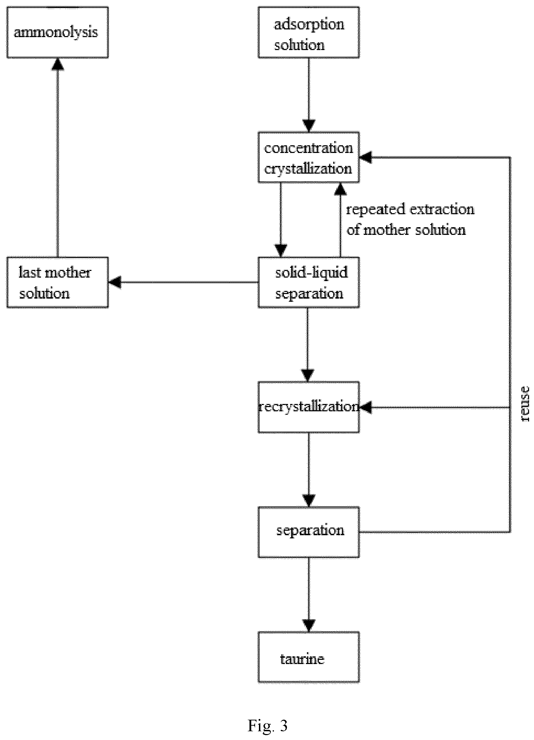

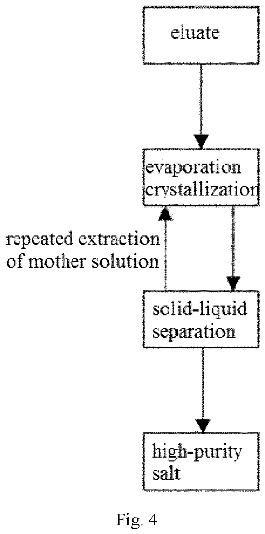

With reference to Fig. 1 to Fig. 4, a method for preparing taurine and salt includes the following steps S1 to S5:
S1: ethylene oxide reacts with a bisulfite solution to generate isethionate; wherein preferred reaction system process conditions are that: the concentration of the bisulfite solution is 25wt% to 60wt%, a molar ratio of bisulfite to the ethylene oxide is (1.03 to 1.08):1, a temperature is 50 DEG C to 80 DEG C, and pH is 6 to 7.
S2: the isethionate obtained in the step S1 is mixed with ammonia liquor and a metal salt to obtain a reaction solution, wherein with respect to a mixing ratio, if an amount of substance of the isethionate is 1mol, an amount of substance of ammonia is greater than 14mol, and an amount of substance of the metal salt is 0.01mol to 0.3mol. After the ammonia is adsorbed until the ammonia content is greater than 20%g/ml, an ammonolysis reaction is performed, and after the reaction is completed, residual ammonia gas is recycled as a raw material of the ammonolysis reaction. A feed solution obtained after the ammonolysis reaction is completed is subjected to evaporation concentration to obtain a concentrated solution.

Wherein, the ammonolysis reaction is carried out at 150 DEG C to 290 DEG C, preferably 240 DEG C to 260 DEG C under a pressure of10MPa to 25MPa for 15min to 60min preferably 40min to 50min.

If a last taurine mother solution is reused in the ammonolysis process, a use amount of the metal salt can be more, and if the last taurine mother solution is not reused, the use amount of the metal salt can be reduced. Specifically, when the reaction solution is the isethionate, the ammonia and the metal salt, a preferred molar ratio is 1:(14 to 17):(0.01 to 0.05); and when the reaction solution is the isethionate, the ammonia, the metal salt and the reused last mother solution, a preferred molar ratio of isethionic acid metal salt to the ammonia to the metal salt is 1:(14 to 17):(0.05 to 0.3). When ammonium isethionate is in use, an adding proportion of the metal salt needs to be properly increased, and preferably, a molar ratio of the ammonium isethionate to the ammonia to the metal salt is 1:(14 to 17):(1.01 to 1.3).

S3: the concentrated solution obtained in the step S2 is prepared to be a concentration of 10% to 35% (in terms of the taurine) and preferably a concentration of 10% to 25%. A solution added in the preparation process may be purified water and reused cleaning water discharged from an adsorption unit of an ion exchange system.

The concentrated solution is sequentially subjected to adsorption, water cleaning, elution and water cleaning treatment by the ion exchange system. An adsorbent of the ion exchange system preferably is resin; the resin is resin with a function that cations can be adsorbed, and includes cation exchange resin such as cation exchange resin with a carboxylic group, a sulfonic group, a phosphate group, a phenolic group and the like, etc.; and meanwhile, chelate resin with the function of adsorbing the cations can also be used.

Metal ions are adsorbed by the resin, and after adsorption, when pH of a solution at an outlet of the adsorption unit is 4 to 10, the adsorption solution is collected. In the adsorbing process, part of the cations is allowed to penetrate through, i.e., the metal cations which are at least equivalent in amount to isethionate anions are allowed to penetrate through to enter the adsorption solution, and the content of the metal cations in the adsorption solution is 0.05% to 2% and preferably is 0.1% to 1%. There is still part of isethionate which is not completely reacted in the concentrated solution, the ion exchange system only adsorbs the cations and the anions can all penetrate through, and thus, it is better to allow the metal ions which are equivalent in amount to the isethionate anions to penetrate through so as to ensure that ingredients at an adsorption solution outlet are the taurine and the isethionate. Otherwise, adsorbing excessive or all the metal ions enables the ingredients at the adsorption solution outlet to exist in a form of the taurine and isethionic acid, and the isethionic acid in the ingredients is relatively unstable and is liable to deteriorate and decompose in the subsequent heating evaporating concentrating process; meanwhile, the isethionic acid is relatively high in viscosity, which can cause big influence on subsequent extraction of the taurine; and in addition, the isethionic acid belongs to medium strong acid, and under the superacid condition, a crystalline state of the taurine is poor, resulting in influence on subsequent extraction of the taurine and reduction of the purity of the taurine.

The purified water is added into the ion exchange system, a use volume of the purified water generally is one to two times of a volume of a resin column of the ion exchange system, and the adsorption unit is cleaned. Cleaning is carried out until the content of the taurine cannot be detected out, and the cleaning water is collected. The cleaning water is reused in the concentrated solution after the ammonolysis reaction.

An acid used as an eluting agent is added into the ion exchange system, the metal ions which have been adsorbed in the resin are continuously eluted. The acid may be sulfuric acid, hydrochloric acid, phosphoric acid, water-soluble carboxylic acid, sulfonic acid and the like. The concentration of the acid entering the ion exchange is 5% to 35% and preferably is 15% to 25%. Only the cations are adsorbed in the ion exchange system, and thus, the eluted metal ions are relatively high in purity so as to ensure that a salt solution with a very high purity is collected. Specifically, if the acid is the sulfuric acid, the correspondingly obtained salt is sulfate, if the acid is the hydrochloric acid, the correspondingly obtained salt is chloride, and the sulfate and the chloride have high solubility in the water. The eluate can be collected when pH of the solution at an outlet of the elution unit is 2 to 8.

The elution unit is cleaned by the purified water, a use volume of the purified water generally is one to two times of a volume of the resin column of the ion exchange system. The cleaning water obtained after water cleaning is collected and is reused in the salt extraction process.

A cleaning mode is adopted for both the adsorption and elution units, and the cleaning process can be implemented by adopting an automatic control system to carry out online operation, so that complete isolation between adsorption and elution can be implemented, thereby ensuring complete isolation between a taurine solution in the adsorption solution and the salt solution in the eluate. The cleaning water can be recycled into corresponding material systems according to ingredients of included materials.

A principle of ion exchange to which the present disclosure relates will be illustrated as follows:
in the ion exchange system, by utilizing the property that the ion exchange resin only adsorbs the cations, the ion exchange resin is used for adsorbing the metal cations in a material (the concentrated solution entering the ion exchange system); in the adsorbing process, H⁺ is exchanged into the material so as to implement separation of the metal cations in the material; after adsorption is completed, by water cleaning, it is guaranteed that there is no residual adsorption solution material in the ion exchange resin; and the obtained cleaning water can be reused in a production process system. The resin after cleaning contains a great amount of metal cations, and does not contain other ingredients. The metal cations in the resin are eluted by the acid, the resin is recycled into an acid state, and a corresponding aqueous solution of the metal salt is collected. After elution is ended, residual acid and salt are cleaned up by water cleaning. After the ion exchange is carried out, the aim of independently separating the taurine and the salt in two material systems is finally fulfilled so as to respectively carry out purification on the taurine and the salt. In comparison, in the original conventional process, the taurine and the salt are mixed and the two products are extracted in the same material system.

Illustration will be further carried out in detail below by taking a process of carrying out ammonolysis to generate sodium taurate and generating the sodium sulfate after adding the sulfuric acid as an example.

*H*₂*NCH*₂*CH*₂*SO*₃*⁻Na*⁺+ *R⁻H*⁺ → *H*₂*NCH*₂*CH*₂*SO*₃⁻*H*⁺ +*R⁻Na*⁺

*H*₂⁺*SO*₄^{2*-*} + 2*R*⁻*Na*⁺ → *Na*₂⁺*SO*₄^{2*-*} +2*R⁻H*⁺

After the ammonolysis reaction is performed on a reaction solution obtained after sodium hydroxyethyl sulfonate, sodium hydroxide and ammonia liquor are mixed, a main ingredient in the reaction solution is the sodium taurate, and the reaction solution also contains part of excessive sodium hydroxide, a little sodium hydroxyethyl sulfonate which is not completely reacted, sodium taurate derivatives and the like; when the ammonolysis reaction is completed and the ammonia is recycled, by the ion exchange system, a flow rate and pH are controlled so as to enable sodium ions to be adsorbed according to a certain proportion; and meanwhile, it has to be ensured that part of residual sodium ions penetrate through the adsorption solution, so that the main ingredients of the collected adsorption solution are the taurine and the sodium hydroxyethyl sulfonate. The sodium hydroxyethyl sulfonate is very high in solubility, and due to very low content of the sodium hydroxyethyl sulfonate in the system, the sodium hydroxyethyl sulfonate cannot be separated out, so that an extraction rate of the taurine will not have the problem caused by influence of separation-out of the sodium sulfate in the conventional process. Furthermore, due to the high solubility of the sodium hydroxyethyl sulfonate, when solid-liquid separation is carried out, a little residual sodium hydroxyethyl sulfonate on the surface of the taurine can be very easily washed off in a water cleaning mode, and thus, the purity of the taurine product is also correspondingly improved. Thus it can be seen that according to the present disclosure, the method implements complete separation between a taurine solution system and a sodium sulfate solution system by the ion exchange system, is an efficient and simple separation method, and meanwhile, also fulfills the aim of preparing the high-purity taurine and sodium sulfate.

S4: the adsorption solution collected in the step S3 is separately collected, and extraction treatment of the taurine is carried out. In the extraction treatment, a collected adsorption solution material can be subjected to evaporation concentration, cooling crystallization and solid-liquid separation by adopting the prior art. In this implementation, preferably, the content for evaporation concentration is 25% to 40% (in terms of the taurine), the cooling crystallization is carried out at 5 DEG C to 30 DEG C, the solid-liquid separation is carried out at5 DEG C to 30 DEG C, preferably 15 DEG C to 20 DEG C, a taurine crude product and a mother solution are obtained, and the taurine crude product has a content of over 95% and the purity of 98.5% or above. The mother solution can be repeately concentrated and crystallized to extract the taurine so as to further improve the extraction rate. Wherein, main ingredients of the mother solution in a taurine extraction system are the taurine and the isethionic acid metal salt. The last mother solution can be recycled as a raw material directly or after impurity removal treatment, and a preferred solution is that the last mother solution is returned into the step S2 to participate in the ammonolysis reaction. As a preferred solution, water and activated carbon are added into the obtained taurine crude product to be subjected torecrystallization, and after solid-liquid separation, a finished product taurine with a higher purity is obtained. In order to better carry out recycling, the mother solution after recrystallization purification can be returned to the initial stage of carrying out evaporation concentration on the adsorption solution in the step S4, and then concentration crystallization and separation are carried out.

According to a specific implementation solution, the taurine crude product recrystallization process is that the taurine crude product is dissolved with 2 to 3 times of purified water, the activated carbon of which the mass is 0.1% to 0.3% of total mass of the crude product is added, decoloration is carried out for 15min to 40min at a temperature of 85 DEG C to 98 DEG C, and after the activated carbon is filtered, the filtrate is cooled to a temperature of 5 DEG C to 30 DEG C and preferably 10 DEG C to 20 DEG C to be subjected to crystallization. Then a crystallized material is dried after being separated so as to obtain the high-purity finished product taurine.

S5: the material of the eluate collected in the step S3 is allowed to enter the extraction treatment process of the salt, and any one applicable prior art can be adopted. In this embodiment, it is preferred to directly extract the salt by adopting an evaporation crystallization process, and then solid-liquid separation is carried out, wherein the evaporation crystallization is preferably carried out at 60 DEG C to 125 DEG C preferably 85 DEG C to 110 DEG C, after the eluate is crystallized, the solid-liquid separation is carried out at60 DEG C to 100 DEG C, preferably 85 DEG C to 95 DEG C, and the sulfate obtained by separation has a content of over 97% and a purity of 98.5% or above. In order to improve the purity of the salt, the obtained salt also can be recrystallized.

The method for producing the high-purity taurine and salt, provided by the present disclosure, can be carried out in a discontinuous, semi-continuous and continuous mode.

Certainly, as a selection, the eluate collected in the step S3 can also be directly used as a high-purity sulfate solution.

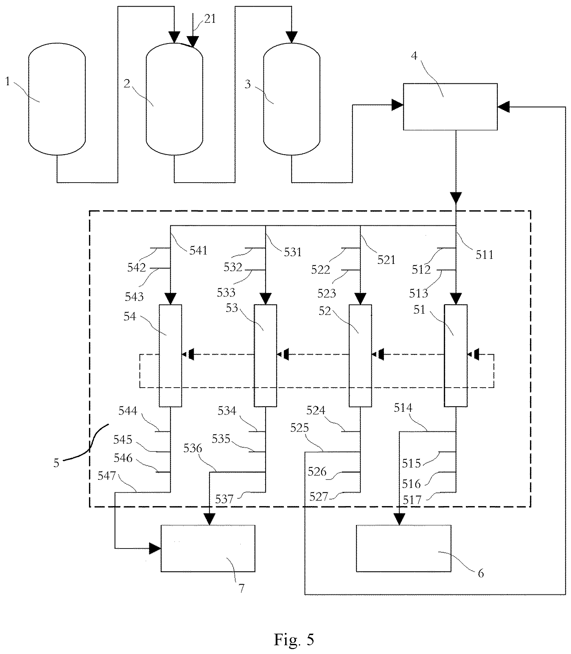

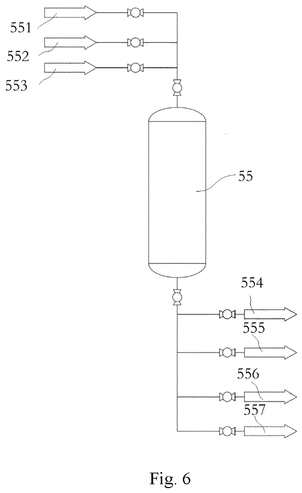

According to the process method above, the disclosure provides a corresponding production system for preparing the high-purity taurine and salt. As shown in Fig. 5 and Fig. 6, the production system includes an addition reaction device 1 of the ethylene oxide and the bisulfate, an ammonolysis reaction device 2, an evaporation device 3 and a taurine salt concentrated solution collection device 4; the taurine salt concentrated solution collection device 4 is connected with an ion exchange system 5; and a concentrated solution enters the ion exchange system 5 to be subjected to ion exchange. Each device and system above are all provided with material inlets and outlets and control valves, which are required for process demands, wherein the ammonolysis reaction device 2 is provided with a material inlet 21 for the metal salt, an ammonia liquor and reused ammonia gas inlet and an inlet for receiving a material discharged by the addition reaction device 1.

The present disclosure can select any one existing ion exchange system, a structure of the ion exchange system is not limited, may be a fixed bed, or may also be a pulse bed or a simulated moving bed which combines a continuous countercurrent system technology on the basis of a conventional fixed bed resin adsorption and ion exchange process, or may be a continuous exchange bed. An existing continuous exchange bed includes a plurality of resin columns, and can complete the whole technological processes of adsorption, elution and water cleaning in one process cycle. In comparison, the fixed bed carries out operations of all the steps in an intermittent process in a mode that each step is carried out for a period of time.

In this embodiment, the ion exchange system includes four cation adsorption resin columns 51, 52, 53 and 54, and an electromagnetic pump. Each resin column is provided with three material inlets and four material outlets, and entrance and exit of the materials are controlled by the valves. The three material inlets include concentrated solution inlets 511, 521, 531 and 541, acid inlets 512, 522, 532 and 542and purified water inlets 513, 523, 533 and 543, and the material outlets include adsorption solution outlets 514, 524, 534 and 544, adsorption cleaning water outlets 515, 525, 535 and 545, eluate outlets 516, 526, 536 and 546 and elution cleaning water outlets 517, 527, 537 and 547, wherein the adsorption solution outlets 514, 524, 534 and 544 are connected with a taurine extraction device 6, and the eluate outlets 516, 526, 536 and 546 are connected with a salt extraction device 7. The adsorption cleaning water outlets 515, 525, 535 and 545 are connected with the concentrated solution collection device 4, and the elution cleaning water outlets 517, 527, 537 and 547 are connected with the salt extraction device 7. The material inlets and the material outlets of the resin columns are all fixed, three material inlets can share one inlet, four material outlets can share one outlet, and entrance and exit switching of each material is implemented by a valve array or in any one of other feasible valve control modes. When the system runs, in the same time period, four resin columns are respectively in different process states and simulate a continuous ion exchange system running mode.

Fig. 5 shows working states of four resin columns 51, 52, 53 and 54 in the same time period, in which processes of adsorption, water cleaning, elution and water cleaning are sequentially carried out. Specifically, the resin column 51 is in an adsorbing state, the valve of the concentrated solution inlet 511 is opened, the concentrated solution in the concentration solution collection device 4 flows into the resin column 51, the valves of the acid inlet 512 and the purified water inlet 513 are closed, at a material outlet end, the valve of the adsorption solution outlet 514 is opened, the taurine extraction device 6 collects the adsorption solution, and the valves of the adsorption cleaning water outlet 515, the eluate outlet 516 and the elution cleaning water outlet 517 are closed; the resin column 52 is in a water cleaning state, the valve of the purified water inlet 523 is opened, purified water is introduced into the resin column 52, the valves of the concentrated solution inlet 521 and the acid inlet 522 are closed, at the material outlet end, the valve of the adsorption cleaning water outlet 525 is opened, cleaning water flows into the concentrated solution collection device 4, and the valves of the adsorption solution outlet 524, the eluate outlet 526 and the elution cleaning water outlet 517 are closed; the resin column 53 is in an eluting state, the valve of the acid inlet 532 is opened, acid is introduced into the resin column 53, the valves of the purified water inlet 533 and the concentrated solution inlet 531 are closed, at the material outlet end, the eluate outlet 536 is opened, the eluate flows into the salt extraction device 7, and the valves of the elution cleaning water outlet 537, the adsorption solution outlet 534 and the adsorption cleaning water outlet 535 are closed; and the resin column 54 is in a water cleaning state, the valve of the purified water inlet 543 is opened, the purified water is introduced into the resin column 54, the valves of the concentrated solution inlet 541 and the acid inlet 542 are closed, at the material outlet end, the valve of the elution cleaning water outlet 547 is opened, elution cleaning water flows into the salt extraction device 7, and the valves of the adsorption solution outlet 544, the eluate outlet 546 and the adsorption cleaning water outlet 545 are closed. Besides the equipment above, the system further includes a control system, power equipment, a connection part, a pipeline valve and the like which are necessary for production, are all common technical means in the art and are not repeated herein. In the processes of adsorption, elution and twice water washing of the ion exchange system, separate collection of an adsorption unit and an elution unit on outlet materials can be implemented by the valve array control system, and the outlet materials include the adsorption solution, the eluate and the cleaning water discharged from the adsorption unit and the elution unit.

pH values of the outlet solution are acquired by pH value monitoring devices arranged in the outlet of the adsorption unit and the outlet of the elution unit in the ion exchange system, and discharge time of the adsorption solution and the eluate are selected so as to collect an aqueous solution containing a high-purity taurine adsorption solution material and the high-purity salt.

The taurine extraction device 6 and the salt extraction device 7 can adopt any applicable equipment and system in the prior art, and for example, existing evaporation equipment, cooling crystallization equipment/crystallization equipment and solid-liquid separation equipment are utilized to constitute the extraction device. The solid-liquid separation equipment in the extraction device can select any applicable existing equipment, such as a panel centrifuge, a top-suspended centrifuge, a continuous horizontal spiral centrifuge, a plate-and-frame filter press and the like.

As shown in Fig. 6, as a selection, if only one resin column 55 is arranged in the ion exchange system, similarly, the resin column 55 includes three material inlets 551, 552 and 553 and four material outlets 554, 555, 556 and 557, the three material inlets can share one inlet, the four material outlets can share one outlet, the ion exchange system is controlled by the valve to sequentially proceed to different working states of adsorption, water cleaning, elution and water cleaning in an intermittent moving mode.

In order to illustrate the technical effects of the preparation method disclosed by the present disclosure, the following examples are used for illustration. Unless otherwise specified, raw materials used in the following embodiments are all commercially available products; unless otherwise specified, methods used in the following embodiments are all conventional methods; and unless otherwise specified, the material content all refers to percentage by mass volume.

### Embodiment 1

This embodiment shows a preparation process of an ammonolysis solution.

Preparation of the sodium hydroxyethyl sulfonate is that: the sodium bisulfate and the ethylene oxide are mixed according to a molar ratio of the sodium bisulfate to the ethylene oxide of 1.05:1, and the mixture is subjected to a reaction under the conditions of a pH value of 6.2 to 6.8 and a temperature of 60 DEG C to 65 DEG C so as to prepare the sodium hydroxyethyl sulfonate.

Preparation of the ammonolysis solution is that: the sodium hydroxyethyl sulfonate, sodium hydroxide and the ammonia liquor are subjected to a reaction for 45min under the conditions of a temperature of 255 DEG C and a pressure of 10MPa to 18MPa, after the reaction is completed, the ammonia gas is discharged and recycled, and concentration is carried out to obtain the ammonolysis reaction concentrated solution.

### Embodiment 2

This embodiment shows that the ion exchange system adsorbs the sodium ions and implements separation into two material solution systems of the taurine and the sodium sulfate.

The ammonolysis concentrated solution is prepared into 18L of solution in which the taurine content is 20%. 12L of sulfuric acid solution with a concentration of 15% is prepared. In a small-sized ion exchange system with resin with the sodium ion adsorption function, the prepared taurine solution is pumped into the system, pH of the collected adsorption solution is controlled within a range of 7 to 8, the content of the sodium ions in the adsorption solution is 0.05% to 1%, 22L of adsorption solution is collected in total, the taurine content is 16%, and the sodium hydroxyethyl sulfonate content is 1.5%. The system is cleaned with purified water, the cleaning water of the adsorption unit is reused in a material of the concentrated solution, and cleaning is carried out until the taurine content cannot be detected out. The sulfuric acid solution with a concentration of 15% is pumped into the cleaned system, pH of the collected eluate is controlled within a range of about 3 to 5, and 16L of eluate of which sodium sulfate content is 17% is collected in total. After collection is completed, the system is cleaned with the purified water, and the cleaning water of the elution unit is discharged, so that a cyclic adsorbing and separating function can be achieved. The system can implement continuous feeding and discharging control by adopting a plurality of resin columns, and meanwhile, ensures stability of the material content of different collected solutions.

| Items | Adsorption Solution | Eluate |
|---|---|---|
| Taurine Content (g/ml) | 16% | smaller than 1ppb |
| Sodium Hydroxyethyl Sulfonate Content (g/ml) | 1.50% | smaller than 1PPb |
| Sodium Sulfate Content (g/ml) | smaller than 10PPm | 17% |

### Embodiment 3

This embodiment shows a process of respectively carrying out concentration extraction on the adsorption solution and the eluate which are collected in Embodiment 2.
(1) Extraction of the adsorption solution is that: 3L of adsorption solution is taken and concentrated into the taurine content of 33%, and cooling is carried out to a temperature of 15 DEG C to carry out centrifugal separation so as to obtain a taurine crude product with the content of 95.5%(g/g) and moisture content of 3.5%(g/g). When moisture is removed, the content is 98.96%(g/g), and the purity is very high. After the taurine is extracted, in the mother solution, the taurine content is 10%, the sodium hydroxyethyl sulfonate content is 4.7%, and the sulfate content is smaller than 10PPm.
(2) Extraction of the eluate is that: 3L of eluate is taken, subjected to six-time concentration crystallization at a high temperature of 95 DEG C and subjected to centrifugal separation to obtain sodium sulfate with the content of 97.5%(g/g) and the moisture content of 2.0%(g/g). After the moisture is removed, the content is 99.49%(g/g), and the purity is very high. In the mother solution, the taurine content is smaller than 1ppb, the sodium hydroxyethyl sulfonate content is smaller than 1ppb, and the sulfate content is 35%.

| | Extraction of Taurine from Adsorption Solution | Extraction of Sodium Sulfate from Eluate |
|---|---|---|
| Taurine Content (g/g) | 95.50% | <1ppb |
| Sodium Hydroxyethyl Sulfonate Content (g/g) | <0.05% | <1ppb |
| Sodium Sulfate Content (g/g) | <10ppm | 97.50% |
| Moisture Content(g/g) | 3.50% | 2.00% |
| Purity After Removal of Moisture | 98.96% | 99.49% |

| | Mother Solution after Extraction of Taurine | Mother Solution after Extraction of Sodium Sulfate |
|---|---|---|
| Taurine Content (g/ml) | 10% | <1ppb |
| Sodium Hydroxyethyl Sulfonate Content (g/ml) | 4.70% | <1ppb |
| Sodium Sulfate Content (g/ml) | <10PPm | 35% |

### Embodiment 4

This embodiment shows a process of respectively carrying out recrystallization on the taurine and the sodium sulfate.

Recrystallization of the taurine is that: 200g of the taurine crude product collected in Embodiment 3 is taken, 500g of purified water is added, 0.4g of activated carbon is added, heating is carried out to a temperature of 95 DEG C, heat preservation is carried out for 20min, after the activated carbon is filtered, cooling is carried out to a temperature of 15 DEG C to carry out crystallization, the crystallization solution is separated, and after drying, 155g of dry fine product taurine is obtained; and the fine mother solution obtained by separation can be recycled and reused in the previous process which may be the concentration crystallization process, or can be returned into a next batch of materials in the recrystallization process. Detected data is as follows:

| Items | High-Purity Fine Product Taurine |
|---|---|
| Appearance: | white crystal particles, no odor and tartish taste |
| Content(C2H7NSO3): | 99.9% |
| Chloride PPM: | <10ppm |
| Sulfate PPM: | <10ppm |
| Ammonium Salt PPM: | <10ppm |
| Residues on Ignition: | 0.005% |
| Loss on Drying: | 0.05% |
| Heavy Metal (in terms of Pb): | <1ppm |
| Arsenic Salt (in terms of As): | <1ppm |

Recyrstallizsation of the sodium sulfate is that: 200g of sodium sulfate collected in Embodiment 3 is taken, 500g of purified water is added, heating is carried out to a temperature of 95 DEG C to carry out evaporation crystallization, the crystallization solution is separated, and after drying, 190g of dry fine product sodium sulfate is obtained; and the mother solution obtained by separation can be recycled and reused in the previous process, i.e., the concentration crystallization process, or can be returned into a next batch of materials in the recrystallization process.

Detected data is as follows:

| Items | High-Purity Fine Product Sodium Sulfate |
|---|---|
| Appearance: | white crystal particles |
| Content(Na2SO4): | 99.90% |
| Chloride PPM: | <10ppm |
| Moisture Content | 0.02% |
| Heavy Metal (in terms of Pb): | <1ppm |
| Arsenic Salt (in terms of As): | <1ppm |

### Embodiment 5

This embodiment shows a conventional method for producing and separating the taurine and the sodium sulfate.

Extraction of the taurine is that: after the ammonolysis concentrated solution is prepared into a certain concentration, under the stirring condition, concentrated sulfuric acid is slowly added for neutralization, a temperature in the neutralizing process is controlled within a range of 50 DEG C to 60 DEG C, when pH is 8.0, the addition of the acid is stopped, cooling is carried out to a temperature of 33 DEG C to carry out crystallization, and separation is carried out to obtain a taurine crude product and a mother solution; and by detection, in the crude product, the taurine content is 90%(g/g), the sodium sulfate content is 3%(g/g) and the moisture content is 6%, and in the mother solution, the taurine content is 15%, and the sulfate content is 25%.

Extraction of the sodium sulfate is that: the mother solution obtained after the taurine is extracted is concentrated again into a content of 33%, the sodium sulfate is separated at a high temperature of 95 DEG C or above, and in the product obtained by separation, the sodium sulfate content is 90%(g/g), the moisture content is 5%, and the taurine content in the sulfate is 4%. The mother solution subjected to separation is cooled to a temperature of 33 DEG C to extract the taurine, and separation and extraction of the sodium sulfate and the taurine are repeatedly carried out in this way.

| | Extraction of Taurine by Conventional Process | Extraction of Sodium sulfate by Conventional Process |
|---|---|---|
| Taurine Content (g/g) | 90.00% | 4% |
| Sodium Hydroxyethyl Sulfonate Content (g/g) | <0.05% | <0.1% |
| Sodium Sulfate Content(g/g) | 3% | 90.00% |
| Moisture Content (g/g) | 6% | 5.00% |
| Purity After Removal of Moisture | 95.74% | 94.74% |

### Embodiment 6

This embodiment shows that the ion exchange system adsorbs the sodium ions and implements separation into two material solution systems of the taurine and sodium chloride.

The ammonolysis concentrated solution is prepared into 18L of solution in which the taurine content is 20%. 24L of hydrochloric acid solution with a concentration of 12% is prepared. The prepared taurine solution is pumped into the ion exchange system, pH of the collected adsorption solution is controlled within a range of 7 to 8, the content of the sodium ions in the adsorption solution is 0.05% to 1%, 22L of adsorption solution is collected in total, the taurine content is 16%, and the sodium hydroxyethyl sulfonate content is 1.5%. The system is cleaned with purified water, the cleaning water is reused in a material of the ammonolysis concentrated solution, and cleaning is carried out until the taurine content cannot be detected out. The hydrochloric acid solution with a concentration of 12% is pumped into the cleaned system, pH of the collected eluate is controlled within a range of about 3 to 5, and 28L of eluate of which the sodium chloride content is 17% is collected in total. After collection is completed, the system is cleaned with the purified water.

| Items | Adsorption Solution | Eluate |
|---|---|---|
| Taurine Content (g/ml) | 16% | <1ppb |
| Sodium Hydroxyethyl Sulfonate Content(g/ml) | 1.50% | <1PPb |
| Sodium Chloride Content (g/ml) | <10PPm | 17% |

By the embodiments above, it can be obviously seen that in the conventional process, the taurine and the sodium sulfate of the material systems are mixed together, resulting in that the product content of the extracted and separated taurine and sodium sulfate is relatively low, the moisture content is high, the purities are poor, and meanwhile, the process of process control is relatively complex. Preparation according to the method disclosed by the present disclosure essentially solves the problem of a case that the taurine and sodium sulfate/sodium chloride material systems are mixed together to be subjected to extraction and separation, and fulfills the aim of preparing the high-purity taurine and sodium sulfate/sodium chloride.

## Claims

1. A method for preparing high-purity taurine and salt, comprising the step of reacting ethylene oxide with bisulfite to generate isethionate, further comprising the steps of carrying out an ammonolysis reaction on the isethionate and ammonia as well as a metal salt, carrying out evaporation to obtain a taurine salt concentrated solution, subjecting the concentrated solution to ion exchange in an ion exchange system to obtain an adsorption solution with a main ingredient of taurine, separately collecting the adsorption solution, and extracting the taurine from the adsorption solution; and eluting adsorbed metal cations by an acid, separately collecting the eluate, and extracting the salt from the eluate or directly using the eluate as a salt solution product, wherein in the ion exchange system, an adsorption unit and an elution unit of the ion exchange system are respectively cleaned with purified water, and correspondingly obtained cleaning water is respectively collected; wherein in an adsorbing state, when pH of a solution at an outlet of the adsorption unit is 4 to 10, the adsorption solution is collected; and in an eluting state, when pH of a solution at an outlet of the elution unit is 2 to 8, the eluate is collected; and wherein metal cations which are at least equivalent in amount to residual isethionate anions are allowed to enter the adsorption solution.

2. The method for preparing the high-purity taurine and salt according to claim 1, wherein in terms of the taurine, the concentration of the concentrated solution entering the ion exchange system is 10% to 35%.

3. The method for preparing the high-purity taurine and salt according to claim 2, wherein the bisulfite is sodium bisulfite, ammonium bisulfite, potassium bisulfite or lithium bisulfite.

4. The method for preparing the high-purity taurine and salt according to claim 3, wherein the metal salt is any one or a mixture of any two or more of sodium hydroxide, potassium hydroxide, lithium hydroxide, sodium carbonate, potassium carbonate, lithium carbonate, sodium sulfate, potassium sulfate and lithium sulfate.

5. The method for preparing the high-purity taurine and salt according to claim 4, wherein the acid is sulfuric acid, hydrochloric acid, phosphoric acid, water-soluble carboxylic acid or sulfonic acid.

6. The method for preparing the high-purity taurine and salt according to claim 5, wherein the concentration of the acid added into the ion exchange system is 5% to 35%.

7. The method for preparing the high-purity taurine and salt according to any one of claims 1 to 6, wherein an adsorbent of the ion exchange system is ion exchange resin; and the ion exchange resin is resin with a function that the cations can be adsorbed.

8. The method for preparing the high-purity taurine and salt according to claim 7, wherein the taurine extraction process of the adsorption solution comprises the steps of: evaporation concentration, cooling crystallization and solid-liquid separation, the cooling crystallization and solid-liquid separation are carried out at 5 DEG C to 30 DEG C, and a taurine crude product obtained by separation has a content of over 95% and a purity of 98.5% or above.

9. The method for preparing the high-purity taurine and salt according to claim 8, wherein the salt is extracted from the eluate in an evaporation crystallization mode, the evaporation crystallization is carried out at 60 DEG C to 125 DEG C, and a salt crude product obtained by separation has a content of over 97% and a purity of 98.5% or above.

10. A system for preparing high-purity taurine and salt, being used for a process for producing the taurine by an ethylene oxide method, comprising an addition reaction device, an ammonolysis reaction device, an evaporation device and a taurine salt concentrated solution collection device, wherein the ammonolysis reaction device is provided with a metal salt inlet, the taurine salt concentrated solution collection device is connected with an ion exchange system for ion exchange, the ion exchange system is provided with an acid inlet, an adsorption solution outlet and an eluate outlet, the adsorption solution outlet is connected with a taurine extraction device, and the eluate outlet is connected with a salt extraction device; and the ion exchange system is provided with a purified water inlet, an adsorption unit cleaning water outlet and an elution unit cleaning water outlet.

11. The system for preparing the high-purity taurine and salt according to claim 10, wherein the adsorption unit cleaning water outlet is connected with the taurine salt concentrated solution collection device, and the elution unit cleaning water outlet is connected with the salt extraction device.

## Patentansprüche

1. Verfahren zur Herstellung von hochreinem Taurin und Salz, umfassend den Schritt des Umsetzens von Ethylenoxid mit Bisulfit, um Isethionat zu erzeugen, weiterhin umfassend die Schritte aus Durchführen einer Ammonolysereaktion an dem Isethionat und Ammoniak sowie einem Metallsalz, Durchführen einer Eindampfung, um eine konzentrierte Taurinsalzlösung zu erhalten, Unterziehen der konzentrierten Lösung einem Ionenaustausch in einem Ionenaustauschsystem, um eine Adsorptionslösung mit einem Hauptinhaltsstoff aus Taurin zu erhalten, separatem Sammeln der Adsorptionslösung, und Extrahieren des Taurins aus der Adsorptionslösung; und Eluieren adsorbierter Metallkationen mittels einer Säure, separatem Sammeln des Eluats, und Extrahieren des Salzes aus dem Eluat oder direktem Verwenden des Eluats als einem Salzlösungsprodukt, wobei in dem Ionenaustauschsystem eine Adsorptionseinheit und eine Elutionseinheit des Ionenaustauschsystems jeweils mit gereinigtem Wasser gereinigt werden, und das entsprechend erhaltene Reinigungswasser jeweils gesammelt wird; wobei in einem adsorbierenden Stadium, wenn der pH einer Lösung an einem Auslass der Adsorptionseinheit 4 bis 10 beträgt, die Adsorptionslösung gesammelt wird; und in einem eluierenden Stadium, wenn der pH einer Lösung an einem Auslass der Elutionseinheit 2 bis 8 beträgt, das Eluat gesammelt wird; und wobei Metallkationen, die zumindest gleich in der Menge zu verbliebenen Isethionat-Anionen sind, das Eingehen in die Adsorptionslösung gestattet wird.

2. Verfahren zur Herstellung des hochreinen Taurins und Salzes gemäß Anspruch 1, wobei in Bezug auf das Taurin, die Konzentration der konzentrierten Lösung, die in das Ionenaustauschsystem eingeht, 10 % bis 35 % beträgt.

3. Verfahren zur Herstellung des hochreinen Taurins und Salzes gemäß Anspruch 2, wobei das Bisulfit Natriumbisulfit, Ammoniumbisulfit, Kaliumbisulfit oder Lithiumbisulfit ist.

4. Verfahren zur Herstellung des hochreinen Taurins und Salzes gemäß Anspruch 3, wobei das Metallsalz irgendeines oder ein Gemisch ist aus zwei oder mehreren von Natirumhydroxid, Kaliumhydroxid, Lithiumhydroxid, Natriumcarbonat, Kaliumcarbonat, Lithiumcarbonat, Natriumsulfat, Kaliumsulfat und Lithiumsulfat.

5. Verfahren zur Herstellung des hochreinen Taurins und Salzes gemäß Anspruch 4, wobei die Säure Schwefelsäure, Salzsäure, Phosphorsäure, wasserlösliche Carbonsäure oder Sulfonsäure ist.

6. Verfahren zur Herstellung des hochreinen Taurins und Salzes gemäß Anspruch 5, wobei die Konzentration der in das Ionenaustauschsystem eingebrachten Säure 5 % bis 35 % beträgt.

7. Verfahren zur Herstellung des hochreinen Taurins und Salzes gemäß einem der Ansprüche 1 bis 6, wobei ein Adsorbens des Ionenaustauschsystems Ionenaustauscherharz ist; und das Ionenaustauscherharz ein Harz mit einer Funktion ist, dass die Kationen adsorbiert werden können.

8. Verfahren zur Herstellung des hochreinen Taurins und Salzes gemäß Anspruch 7, wobei der Taurinextraktionsprozess der Adsorptionslösung die Schritte umfasst: Eindampfungskonzentrierung, Kühlkristallisierung und Fest-Flüssig-Trennung, die Kühlkristallisierung und Fest-Flüssig-Trennung bei 5 DEG C bis 30 DEG C durchgeführt werden, und ein durch Trennung erhaltenes Rohprodukt von Taurin einen Gehalt von über 95 % und eine Reinheit von 98,5 % oder mehr aufweist.

9. Verfahren zur Herstellung des hochreinen Taurins und Salzes gemäß Anspruch 8, wobei das Salz aus dem Eluat in einem Eindampfungskristallisierungsmodus extrahiert wird, die Eindampfungskristallisierung bei 60 DEG C bis 125 DEG C durchgeführt wird, und ein durch die Trennung erhaltenes Rohprodukt des Salzes einen Gehalt von über 97 % und eine Reinheit von 98,5 % oder mehr aufweist.

10. System zur Herstellung von hochreinem Taurin und Salz, zur Verwendung für ein Verfahren zur Herstellung des Taurins mittels einer Ethylenoxidmethode, umfassend eine Additionsreaktionsvorrichtung, eine Ammonolysereaktionsvorrichtung, eine Eindampfvorrichtung und eine Sammelvorrichtung für konzentrierte Taurinsalzlösung, wobei die Ammonolysereaktionsvorrichtung mit einem Einlass für Metallsalz versehen ist, die Sammelvorrichtung für konzentrierte Taurinsalzlösung mit einem Ionenaustauschsystem für den Ionenaustausch verbunden ist, das Ionenaustauschsystem mit einem Säureeinlass, einem Adsorptionslösungsauslass und einem Eluatauslass versehen ist, der Adsorptionslösungsauslass mit einer Taurinextraktionsvorrichtung verbunden ist, und der Eluatauslass mit einer Salzextraktionsvorrichtung verbunden ist; und das Ionenaustauschsystem mit einem Einlass für gereinigtes Wasser, einem Auslass für Reinigungswasser der Adsorptionseinheit und einem Auslass für Reinigungswasser der Elutionseinheit versehen ist.

11. System zur Herstellung des hochreinen Taurins und Salzes gemäß Anspruch 10, wobei der Auslass für Einigungswasser der Adsorptionseinheit mit der Sammelvorrichtung für konzentrierte Taurinsalzlösung verbunden ist, und der Auslass für Reinigungswasser der Elutionseinheit mit der Salzextraktionsvorrichtung verbunden ist.

## Revendications

1. Procédé de préparation de taurine et de sel de haute pureté, comprenant l'étape de réaction de l'oxyde d'éthylène avec un bisulfite pour produire un iséthionate, comprenant en outre les étapes de réalisation d'une réaction d'ammonolyse sur l'iséthionate et l'ammoniaque ainsi qu'un sel métallique, en réalisant une évaporation pour obtenir une solution concentrée de sel de taurine, en soumettant la solution concentrée à un échange d'ions dans un système d'échange d'ions pour obtenir une solution d'adsorption avec un ingrédient principal de taurine, en collectant séparément la solution d'adsorption, et en extrayant la taurine de la solution d'adsorption ; et en éluant les cations métalliques adsorbés par un acide, en collectant séparément l'éluat et en extrayant le sel de l'éluat ou en utilisant directement l'éluat comme produit de solution saline, dans le système d'échange d'ions, une unité d'adsorption et une unité d'élution du système d'échange d'ions étant respectivement nettoyées avec de l'eau purifiée et l'eau de nettoyage obtenue de manière correspondante étant respectivement collectée ; dans un état d'adsorption, quand le pH d'une solution à une sortie de l'unité d'adsorption est de 4 à 10, la solution d'adsorption étant collectée ; et dans un état d'élution, quand le pH d'une solution d'élution à une sortie de l'unité d'élution est de 2 à 8, l'éluat étant collecté ; et les cations métalliques qui sont au moins équivalents en quantité aux anions iséthionate résiduels étant laissés entrer dans la solution d'adsorption.

2. Procédé de préparation de la taurine et de sel de haute pureté selon la revendication 1, dans lequel en termes de la taurine, la concentration de la solution concentrée entrant dans le système d'échange d'ions est de 10 % à 35 %.

3. Procédé de préparation de la taurine et de sel de haute pureté selon la revendication 2, dans lequel le bisulfite est le bisulfite de sodium, le bisulfite d'ammonium, le bisulfite de potassium ou le bisulfite de lithium.

4. Procédé de préparation de la taurine et de sel de haute pureté selon la revendication 3, dans lequel le sel métallique est l'un quelconque ou un mélange des quelconques deux ou plus de deux de l'hydroxyde de sodium, de l'hydroxyde de potassium, de l'hydroxyde de lithium, du carbonate de sodium, du carbonate de potassium, du carbonate de lithium, du sulfate de sodium, du sulfate de potassium et du sulfate de lithium.

5. Procédé de préparation de la taurine et de sel de haute pureté selon la revendication 4, dans lequel l'acide est l'acide sulfurique, l'acide chlorhydrique, l'acide phosphorique, un acide carboxylique hydrosoluble ou l'acide sulfonique.

6. Procédé de préparation de la taurine et de sel de haute pureté selon la revendication 5, dans lequel la concentration de l'acide ajouté dans le système d'échange d'ions est de 5 % à 35 %.

7. Procédé de préparation de la taurine et du sel de haute pureté selon l'une quelconque des revendications 1 à 6, dans lequel un adsorbant du système d'échange d'ions est une résine d'échange d'ions ; et la résine d'échange d'ions est une résine avec une fonction où les cations peuvent être adsorbés.

8. Procédé de préparation de la taurine et de sel de haute pureté selon la revendication 7, dans lequel le processus d'extraction de taurine de la solution d'adsorption comprend les étapes de : concentration par évaporation, cristallisation par refroidissement et séparation solide-liquide, la cristallisation par refroidissement et la séparation solide-liquide sont réalisées à 5 DEG C à 30 DEG C et un produit brut de taurine obtenu par séparation a une teneur de plus de 95 % et une pureté de 98,5 % ou plus.

9. Procédé de préparation de la taurine et de sel de haute pureté selon la revendication 8, dans lequel le sel est extrait de l'éluat dans un mode de cristallisation par évaporation, la cristallisation par évaporation est réalisée à 60 DEG C à 125 DEG C et un produit brut salin obtenu par séparation a une teneur de plus de 97 % et une pureté de 98,5 % ou plus.

10. Système de préparation de taurine et de sel de haute pureté, qui est utilisé pour produire la taurine par un procédé d'oxyde d'éthylène, comprenant un dispositif de réaction d'addition, un dispositif de réaction d'ammonolyse, un dispositif d'évaporation et un dispositif de collecte de solution concentrée de sel de taurine, le dispositif de réaction d'ammonolyse étant fourni avec une entrée de sel métallique, le dispositif de collecte de solution concentrée de sel de taurine étant connecté avec un système d'échange d'ions pour l'échange d'ions, le système d'échange d'ions étant fourni avec une entrée d'acide, une sortie de solution d'adsorption et une sortie d'éluat, la sortie de solution d'adsorption étant connectée avec un dispositif d'extraction de taurine et la sortie d'éluat étant connectée avec un dispositif d'extraction de sel ; et le système d'échange d'ions étant fourni avec une entrée d'eau purifiée et une sortie d'eau de nettoyage d'unité d'adsorption et une sortie d'eau de nettoyage d'unité d'élution.

11. Procédé de préparation de la taurine et du sel de haute pureté selon la revendication 10, dans lequel la sortie d'eau de nettoyage d'unité d'adsorption est connectée avec le dispositif de collecte de de solution concentrée de sel de taurine et la solution d'eau de nettoyage d'unité d'élution est connectée avec le dispositif d'extraction de sel.
